(19) Europäisches Patentamt
European Patent Office
Office européen des brevets

(11) Veröffentlichungsnummer: **0 520 292 A1**

(12) # EUROPÄISCHE PATENTANMELDUNG

(21) Anmeldenummer: **92110132.5**

(22) Anmeldetag: **16.06.92**

(51) Int. Cl.5: **C07D 205/08**, C09K 19/58, G02F 1/13

(30) Priorität: **19.06.91 DE 4120220**
**25.06.91 DE 4120981**

(43) Veröffentlichungstag der Anmeldung:
**30.12.92 Patentblatt 92/53**

(84) Benannte Vertragsstaaten:
**CH DE FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Scherowsky, Günter, Prof. Dr.**
**Winklerstrasse 18b**
**W-1000 Berlin 33(DE)**
Erfinder: **Junghans, Claas**
**Guerickestrasse 1a**
**W-1000 Berlin 10(DE)**
Erfinder: **Michalski, Britta**
**Habelschwerdtstrasse 7**
**W-1000 Berlin 33(DE)**
Erfinder: **Kaltbeitzel, Anke, Dr.**
**Konrad-Adenauer-Ring 12**
**W-6090 Rüsselheim(DE)**
Erfinder: **Wingen, Rainer, Dr.**
**Brunnenstrasse 1**
**W-6234 Hattersheim am Main(DE)**

(54) **Chirale Azetidinon-Derivate und ihre Verwendung als Dotierstoffe in Flüssigkristallmischungen.**

(57) Azetidinone der allgemeinen Formel I

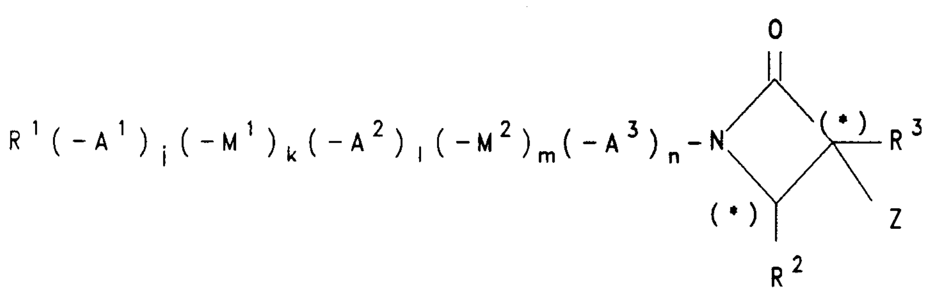

(I)

in der z.B. $R^1$, ein Alkyl oder Alkenylrest, $R^2$, $R^3$ Wasserstoff, ein Alkyl oder Alkenylrest, Z Wasserstoff oder (Pseudo)Halogen, $A^1$, $A^2$, $A^3$ ein Ringsystem und M eine Spacergruppe darstellt, können Flüssigkristallmischungen zugesetzt werden. Sie bewirken schon bei geringen Zumischmengen eine starke Verdrillung in der cholesterischen und der smektischen C*-Phase, wodurch der Pitch eines anderen Dotierstoffs kompensiert werden kann bzw. ein kurzer $S_C$*-Pitch erzeugt werden kann.

EP 0 520 292 A1

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen zu lang. Dieser Nachteil macht sich insbesondere dann bemerkbar, wenn eine große Anzahl von Bildpunkten angesteuert werden muß. Die Herstellungskosten von Geräten, die größere Bildschirmflächen enthalten, wie z.B. von Videogeräten sind dann im allgemeinen zu hoch.

Neben den nematischen und cholesterischen Flüssigkristallen haben seit einigen wenigen Jahren in zunehmendem Maß auch optisch aktive smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu optoelektrischen Schalt-oder Anzeigeelementen führt, die im Vergleich zu den herkömmlichen TN ("twisted nematic")-Zellen um bis zu einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind FLC's grundsätzlich für die obengenannten Anwendungsgebiete, z.B. über eine Matrixansteuerung, gut geeignet.

Für elektrooptische Schalt- und Anzeigeelemente benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S^*_A$ und $S^*_C$-Phase läßt sich erreichen, wenn die Phasenfolge der Flüssigkristallmischung mit abnehmender Temperatur lautet:

$$\text{Isotrop} \rightarrow N^* \rightarrow S^*_A \rightarrow S^*_C$$

Voraussetzung ist, daß der Pitch (Ganghöhe der Helix) in der $N^*$-Phase sehr groß (größer 10 $\mu$m) oder noch besser völlig kompensiert ist (siehe z.B. T. Matsumoto et al., p. 468-470, Proc. of the 6th Int. Display Research Conf., Japan Display, Sept. 30 - Okto. 2, 1986, Tokyo, Japan, M. Murakami et al., ibid. S. 344 - S. 347). Dies erreicht man, indem man zu der chiralen Flüssigkristallmischung, die in der $N^*$-Phase z.B. eine linksdrehende Helix aufweist, einen weiteren optisch aktiven Dotierstoff, der eine rechtsdrehende Helix induziert, in solchen Mengen hinzugibt, daß die Helix gerade kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C* Phase wesentlich größer ist als die Dicke des Anzeigeelementes (Mol. Cryst. Liq. Cryst. 94 (1983), 213-134 und 114 (1984), 151-187). Dies erreicht man wie im Fall des cholesterischen Pitches durch Verwendung von Dotierstoffen mit entgegengesetztem Drehsinn der Helix.

Ferroelektrische Flüssigkristallanzeigen lassen sich auch durch Nutzung des DHF-Effektes oder des PSFLCD-Effektes (Pitch Stabilized Ferroelectric Liquid Crystal Display, auch SBF = Short Pitch Bistable Ferroelectric Effekt genannt) betreiben. Der DHF-Effekt wurden von B.I. Ostrovski in Advances in Liquid Crystal Research and Applications, Oxford/Budapest, 1980, 469 ff. beschrieben, der PSFLCD-Effekt wurde in DE 3 920 625 bzw. EP 0 405 346 A2 beschrieben. Zur Nutzung dieser Effekte wird im Gegensatz zum SSFLCD-Effekt ein flüssigkristallines Material mit einem kurzen $S_C$-Pitch benötigt.

Es wurde nun gefunden, daß optisch aktive Azetidinone als Dotierstoffe in geneigt smektischen Flüssigkristallphasen schon bei geringen Zumischmengen zu einer starken Verdrillung in der cholesterischen und der smektischen $C^*$-Phase führen.

Diese in der $N^*$- und $S_C^*$-Phase induzierte Helix kann in Mischungen vorteilhaft zur gezielten Kompensation der Ganghöhe oder aber zur Erzeugung eines kurzen $S_C$-Pitches verwendet werden. Besonders vorteilhaft ist dabei, daß die erfindungsgemäßen Dotierstoffe aufgrund ihres starken Verdrillungsvermögens schon in geringen Zusatzmengen diese Effekte bewirken.

Die optische Schaltzeit $\tau$ [$\mu$s] ferroelektrischer Flüssigkristallsysteme, die möglichst kurz sein soll, hängt von der Rotationsviskosität des Systems $\gamma$ [mPas], der spontanen Polarisation $P_s$[nC/cm$^2$] und der elektrischen Feldstärke E[V/m] ab nach der Beziehung

$$T \sim \frac{\gamma}{P_s \bullet E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Es wurde überraschend gefunden, daß schon eine geringe Beimengung der erfindungsgemäßen Substanz eine hohe spontane Polarisation in einer nichtchiralen Grundmischung induziert, so daß sich durch den Einsatz dieser Verbindung kurze Schaltzeiten erzielen lassen.

Gegenstand der Erfindung ist daher die Verwendung von chiralen bzw. optisch aktiven Azetidinonen als Dotierstoffe in Flüssigkristallmischungen. Gegenstand der Erfindung sind weiterhin Flüssigkristallsysteme, die chirale bzw. optisch aktive Azetidinone enthalten, sowie neue chirale Azetidinone (sowohl als optisch aktive Verbindungen wie auch als racemische Gemische). Die gemäß der Erfindung einzusetzenden Azetidinone entsprechen der allgemeinen Formel (I)

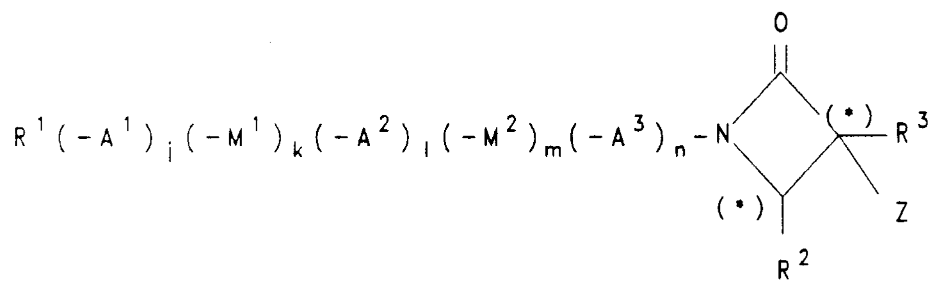

(I)

in der die Symbole und Indices folgende Bedeutung haben:

(*) ist gegebenenfalls ein chirales Zentrum

$R^1$ ist ein geradkettiger oder verzweigter Alkylrest mit 1 bis 16 C-Atomen oder ein geradkettiger oder verzweigter Alkenylrest mit 3 bis 16 C-Atomen, in dem selbst asymmetrische C-Atome enthalten sein können, eine oder mehrere nicht benachbarte $-CH_2$-Gruppen durch -O-, -S-, -CO-, -O-CO- und/oder - CO-O- ersetzt sein können, wodurch sich die Zahl der C-Atome in dem Rest entsprechend reduziert, und in dem ein oder mehrere H durch F, Cl, Br oder CN ersetzt sein können,

$R^2$ ist ein Wasserstoffatom oder ein Alkylrest mit 1 bis 10 C-Atomen, in dem auch die dem Ring benachbarte $-CH_2$-Gruppe durch -O- oder

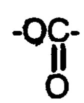

ersetzt sein kann, wodurch sich die Zahl der C-Atome in dem Rest entsprechend reduziert,

$R^3$ ist ein Wasserstoffatom oder ein Alkylrest mit 1 bis 10 C-Atomen

Z bedeutet H, F, Cl, $-NO_2$ oder $-N_3$

j und l sind 0, 1 oder 2,

k und m sind 0 oder 1,

n ist 0, 1 oder 2,

mit folgender Maßgabe: wenn j und/oder l = null sind, ist k = null; wenn n = null ist, ist m = null; die Summe j + l + n ist minimal 1 und maximal 3,

$-A^1$, $-A^2$ bedeutet

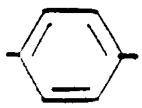 , 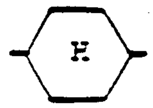 , 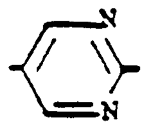 , 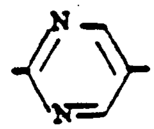 , 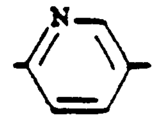 ,

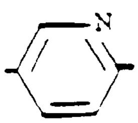 , 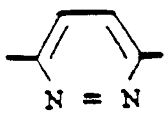 , 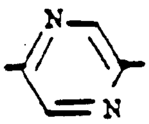 , 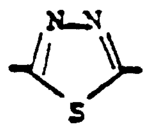 , 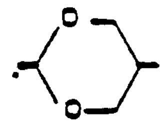 ,

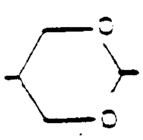 , 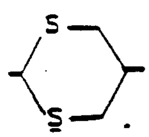 , 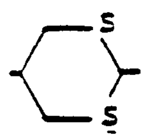 , 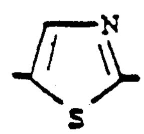 , 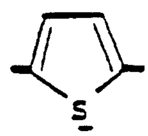 ,

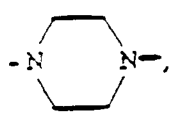 , 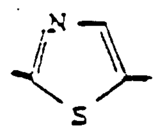 , 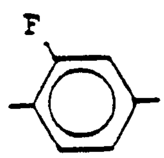 , 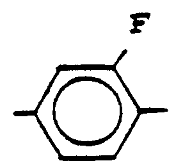 , 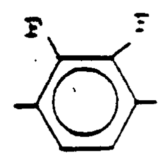 ,

$-A^3$ bedeutet

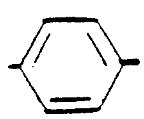 , 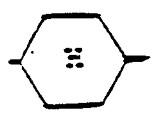 , 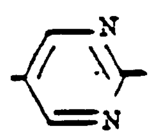 , 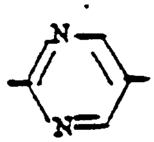 , 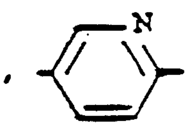 ,

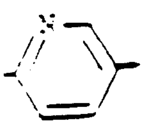 , 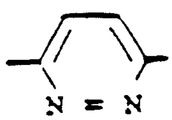 , 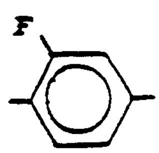 , 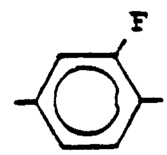 , 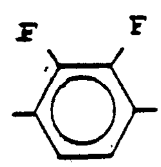

$-M^1$, $-M^2$ ist -O-CO-, -CO-O-, $-CH_2CH_2-$, -CH = CH-, $-CH_2$-O- oder $-O-CH_2-$.

In einer bevorzugten Ausführungsform werden Azetidinone der allgemeinen Formel (I) eingesetzt, in der

$R^1$ ein geradkettiger oder verzweigter Alkyl- oder Alkenylrest mit 6 bis 12 C-Atomen ist, der ein asymmetrisches C-Atom enthalten kann, und in dem eine $CH_2$-Gruppe durch -O-, -CO-, oder -COO- ersetzt sein kann, wodurch sich die Zahl der C-Atome in dem Rest entsprechend reduziert, und

die Gruppierung $(-A^1)_j(-M^1)_k(-A^2)_l(-M^2)_m(-A^3)_n$- folgende Bedeutung hat:

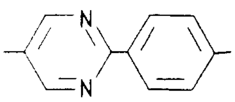 , 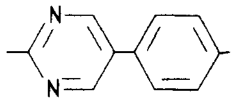 , 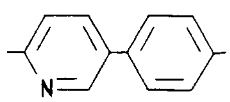 , 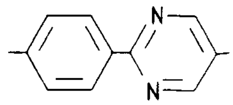

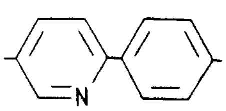 , 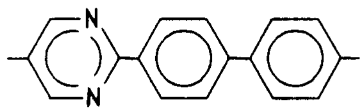 , 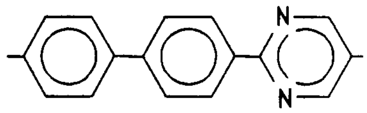 ,

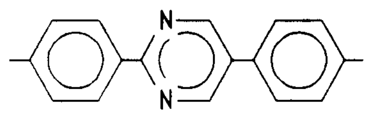 , 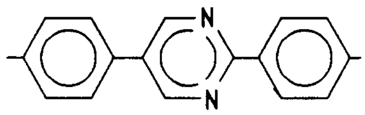 ,

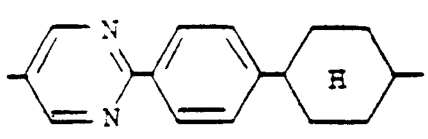 , 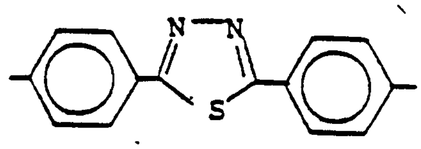

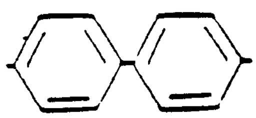 ,

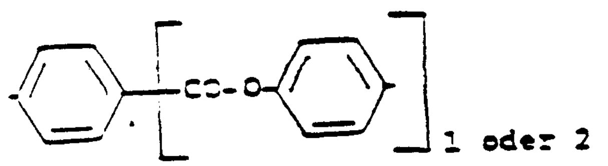 , 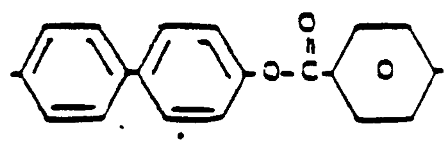

Die Azetidinone der allgemeinen Formel (I) lassen sich auf mehrstufigen Synthesewegen unter Verwendung von literaturbekannten Einzelreaktionen gewinnen. Auf die allgemeinen Darstellungsweisen wird im Experimentellen Teil näher eingegangen.

Die genannten Azetidinone sind als Komponenten für Flüssigkristallmischungen geeignet. Dabei enthalten die LC-Mischungen vorzugsweise 0,01 bis 60 Gew.-%, insbesondere 0,1 bis 20 Gew.-%, besonders bevorzugt 0,1 bis 5 Gew.-% der genannten Azetidinone. Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N, S oder O-haltige Heterocyclen, z.B. Pyrimidine, Zimtsäureester, Cholesterinester oder verschieden überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedener Komponenten vor, von denen mindestens eine mesogen ist, d.h. als Verbindung, in derivatisierter Form oder im Gemisch mit anderen Komponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

Das hohe Verdrillungsvermögen der erfindungsgemäßen Verbindungen führt bereits in nematischen Phasen bei den eher "klassischen" Displaytechnologien zu vorteilhaften Einsatzmöglichkeiten. Hierbei steht jedoch häufig nicht die Kompensation, sondern die Erzielung einer Verdrillung durch eine möglichst geringe Zugabemenge an chiralem Dotierstoff im Vordergrund. Dies gilt sowohl für die TN ("twisted-nematic")-Technologie [siehe M. Schadt et al., Appl. Phys. Lett 18, 127 (1971)] als auch für das sogenannte White-Taylor-Display [D. L. White et al., J. Appl. Phys. 45, 4718 (1974)] oder das SBE/STN ("super-birefringence-effect"/"super-twisted-nematic")-Display [T. J. Scheffer et al., Appl. Phys. Lett. 45, 1021 (1984)] und seine verschiedenen Modifikationen wie das OMI ("optical mode interference") Display [M. Schadt et al., Appl. Phys. Lett. 50, 236 (1987)].

Die Flüssigkristallmischungen können beispielsweise in elektrooptischen Schalt-und Anzeigevorrichtungen eingesetzt werden, die darüber hinaus u.a. folgende Komponenten enthalten: zwei Elektroden, zwei Trägerplatten sowie mindestens eine Orientierungsschicht. Allgemein wird der Aufbau von FLC-Displays in EP-B 0 032 362 beschrieben.

Die Erfindung wird durch die nachfolgenden Beispiele näher erläutert.

Das Verfahren zur Herstellung der Verbindungen der Formel (I) ist dadurch gekennzeichnet, daß nach Schema 1 eine Carbonsäure der Formel (XIII) - die nach im Prinzip literaturbekannten Reaktionen aus bekannten Edukten hergestellt wurde und bei der $P_g$ eine Schutzgruppe, z.B. tert.-Butyldiphenylsilyl oder tert.-Butyldimethylsilyl, bedeutet - mit einem mesogenen Amin (XIX) - hierbei steht $Y^1$ für eine Gruppierung, die mit $Y^2$ im späteren Verlauf $M^2$ ausbilden kann, stellt also z.B. -OH, $-OP_g$, $-CO_2H$ oder $-CO_2R'$ dar - zu einem Anilid der Formel (XIV) umgesetzt wird. (XIV) wird in ebenfalls literaturbekannter Weise durch Entschützung zu (XV) und Funktionalisierung zu (XVI) weiter zum Azetidinon (XVII) umgesetzt. Durch geeignete Reaktion der Funktionalitäten $Y^1$ bzw. $Y^2$ von (XVII) bzw. (XVIII) - z.B. Veresterung - läßt sich (I) erhalten.

Für den Fall, daß m und k jeweils Null bedeuten, $A^3$ und $A^2$ und/oder $A^1$ also durch Einfachbindungen verknüpft sind, kann auch nach Schema 2 die Säure (XIII) mit dem mesogenen Amin (XX) zum Anilid (XXI) umgesetzt werden. Analog zu Schema 1 erfolgen Entschützung (XXII) und Funktionalisierung (XXIII) sowie Ringschluß zu (I).

Schema 1

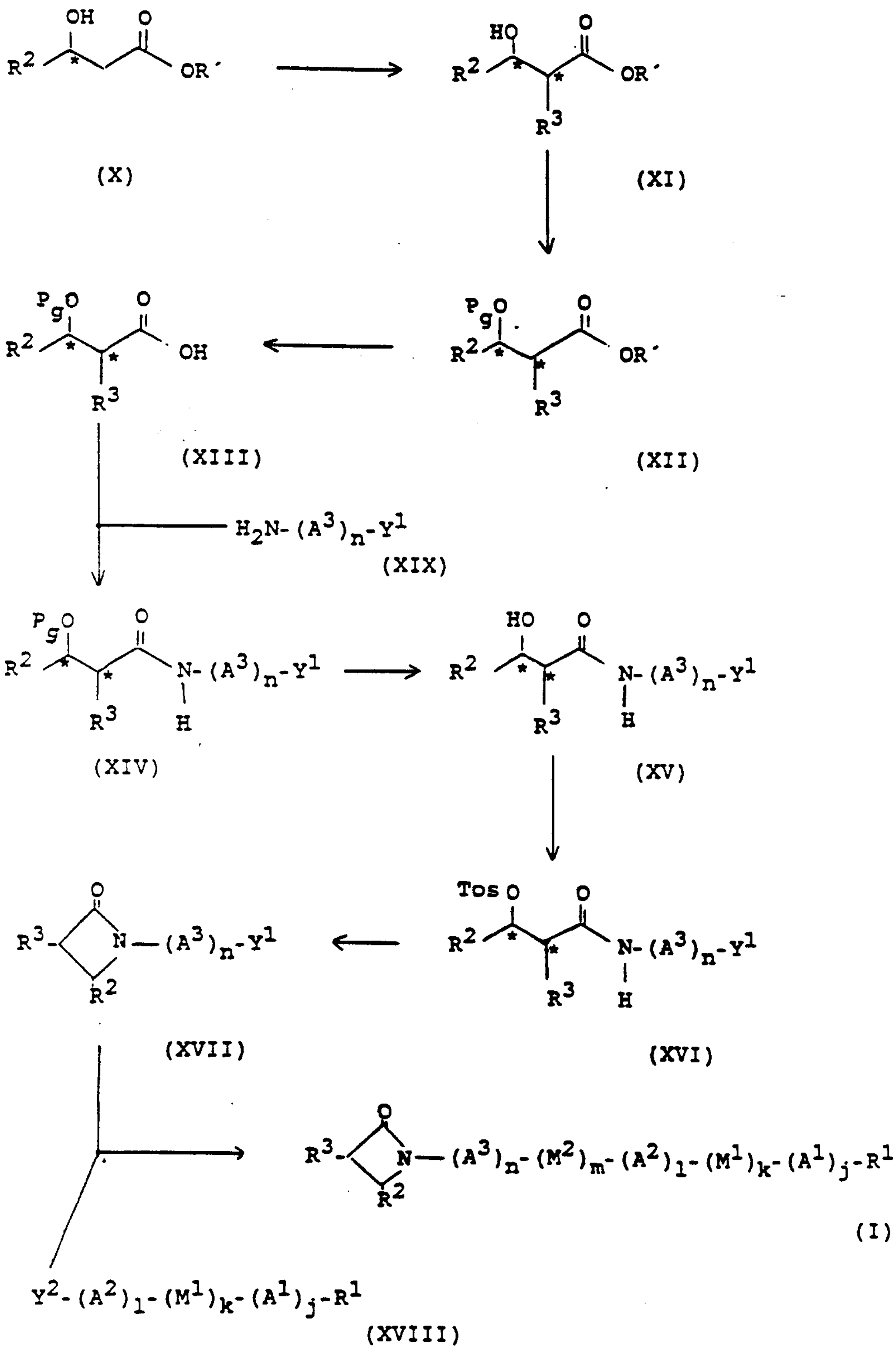

(X) (XI) (XIII) (XII) (XIX) (XIV) (XV) (XVII) (XVI) (I) (XVIII)

Schema 2

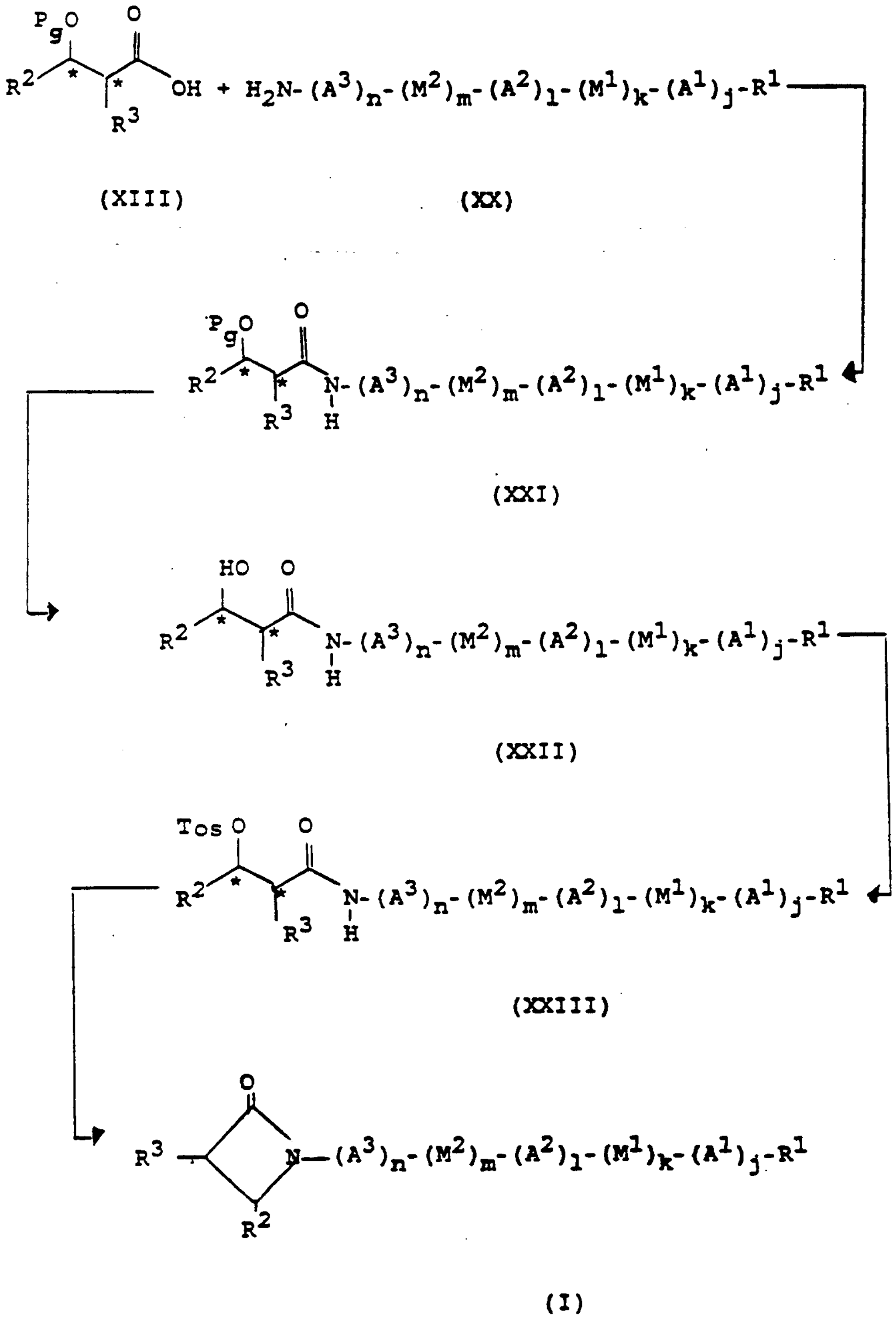

(XIII) (XX)

(XXI)

(XXII)

(XXIII)

(I)

Beispiel 1

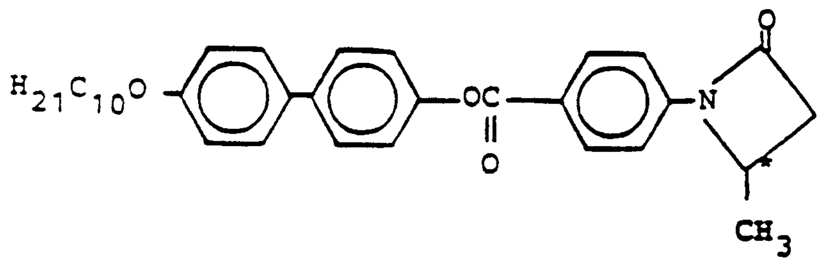

(S)-1-[(4'-Decyloxybiphenyl-4-yl)oxycarbonylphenyl-]4-methyl-azetidin-2-on

18,25 g (R)-3-tert.-Butyldiphenylsilyloxybutansäureethylester - erhalten durch Umsetzung von (R)-3-Hydroxybutansäureethylester mit tert.-Butyldiphenylchlorsilan und Imidazol in Dimethylformamid - werden 70 h mit einer Lösung von 6,4 g Kaliumhydroxid in 100 ml Ethanol bei Raumtemperatur gerührt. Nach Aufarbeitung werden 13,7 g (R)-3-tert.-Butyldiphenylsilyloxybutansäure mit Schmp. 103 ° C und $[\alpha]_D^{20}$ : 1,3 ° (c = 16,9 , $CHCl_3$) erhalten.

Eine Lösung von 4,48 g (R)-3-tert.-Butyldiphenylsilyloxybutansäure in 10 ml Toluol wird mit der doppelt molaren Menge Oxalylchlorid versetzt und 1 h zum Sieden erhitzt. Nach Abdestillation der flüchtigen Komponenten wird in 10 ml Dichlormethan aufgenommen und diese Lösung zu einer Lösung von 2,98 g 4-Aminobenzoesäurebenzylester und 5 ml Triethylamin in 15 ml Dichlormethan getropft. Nach wäßriger Aufarbeitung, Extraktion mit Diethylether und chromatographischer Reinigung (Kieselgel, Hexan/Diethylether 1:1) werden 5,98 g (R)-3-(t-Butyldiphenylsilyloxy)butansäure-(4-benzyloxycarbonyl)anilid als farbloses Öl mit $[\alpha]_D^{20}$ : -2,3 ° (c = 10,9, $CHCl_3$) erhalten.

Zu einer Lösung von 5,17 g (R)-3-(tert.-Butyldiphenylsilyloxy)-butansäure-(4-benzyloxycarbonyl)anilid in 7 ml Tetrahydrofuran werden 18 ml einer 1,1 M Lösung von Tetrabutylammoniumchlorid in Tetrahydrofuran getropft. Nach geeigneter Aufarbeitung und chromatographischer Reinigung werden 1,35 g (R)-3-Hydroxybutansäure-(4-benzyloxycarbonyl)anilid mit Schmp. 114 ° C und $[\alpha]_D^{20}$ : -26,4 ° (c = 9,1, $CHCl_3$) erhalten.

Eine Lösung von 1,02 g (R)-3-Hydroxybutansäure-(4-benzyloxycarbonyl)anilid in 4 ml Pyridin wird mit 1 g 4-Toluolsulfonylchlorid versetzt. Nach üblicher Aufarbeitung und chromatographischer Reinigung werden 1,27 g (R)-3-(4-Methylphenylsulfonyloxy)butansäure-(4-benzyloxycarbonyl)anilid mit Schmp. 87 ° C erhalten.

Zu einer Suspension von 170 mg Natriumhydrid in 60 ml Dichlormethan/Dimethylformamid (4:1) werden binnen 3 h bei 5 ° C 2,44 g (R)-3-(4-Methylphenylsulfonyloxy)butansäure-(4-benzyloxycarbonyl)anilid gegeben. Nach einer weiteren Stunde werden 40 ml gesättigte Ammoniumchloridlösung zugegeben. Die organische Phase wird im Vakuum zur Trockne gebracht, in 50 ml Diethylether aufgenommen und dreimal mit je 20 ml Wasser gewaschen. Nach Eindampfen und Umkristallisation aus Diethylether/Hexan-Gemischen erhält man 1,44 g (S)-1-(4-Benzyloxycarbonylphenyl)-4-methylazetidin-2-on mit Schmp. 89 ° C.

Eine Lösung von 700 mg (S)-1-(4-Benzyloxycarbonylphenyl)-4-methylazetidin-2-on in 25 ml Methanol wird mit Hilfe von 70 mg Pd/C (10 %) hydriert. Nach Aufarbeitung werden 0,455 g (S)-1-(4-Carboxy)phenyl-4-methylezetidin-2-on mit Schmp. 162 ° C erhalten; $[\alpha]_D^{20}$ : 68,2 ° (c = 7,1, $CHCl_3$).

Eine Lösung von 455 mg (S)-1-(4-Carboxy)phenyl-4-methylazetidin-2-on in 3 ml Dichlormethan und 3 ml Dimethylformamid wird mit 750 mg 4-Decyloxy-4'-hydroxybiphenyl sowie 515 mg Dicyclohexylcarbodiimid und 40 mg 4-Dimethylaminopyridin versetzt. Nach üblicher Aufarbeitung und chromatographischer Reinigung werden 350 mg Rohprodukt erhalten, die noch zweimal aus Diethylether/Hexan-Gemischen umkristallisiert werden.

Phasenfolge: $X_1$ 110 $X_2$ 115 $S_x$ 118 N* 174 I

Die folgenden Verbindungen werden analog erhalten bzw. unter Verwendung von (2R, 3R)-2-Octyl-3-hydroxybutansäuremethylester (erhalten aus 3-Hydroxybutansäuremethylester nach G. Fratér, Helv. Chim. Acta 62, 2829 (1979)) und (R)--3-Hydroxy-2-methylpropansäuremethylester.

Beispiel 2

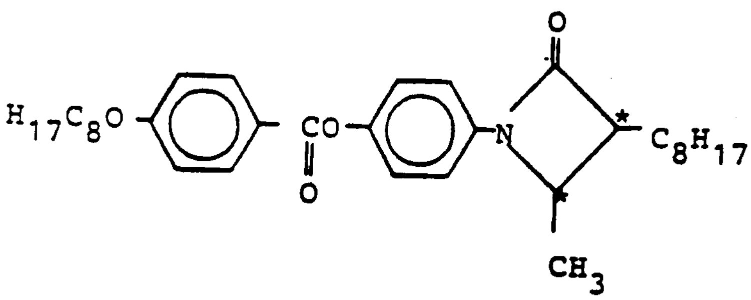

Phasenfolge X 69 $S_C$* 101 $S_A$* 106 N* 128 I

Beispiel 3

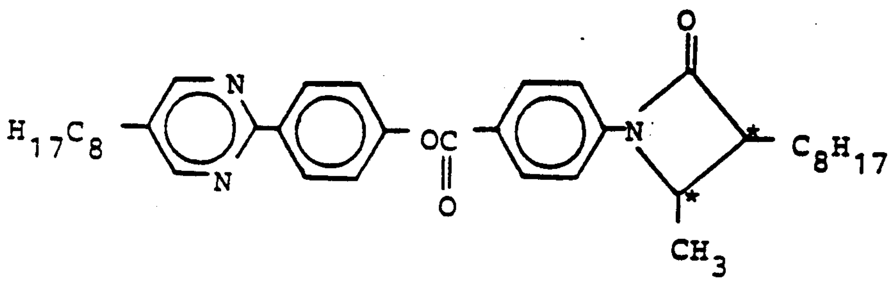

Phasenfolge X 82 N* 212 I $[\alpha]_D^{20}$ : 21,2° (c = 3,4 , $CHCl_3$)

Beispiel 4

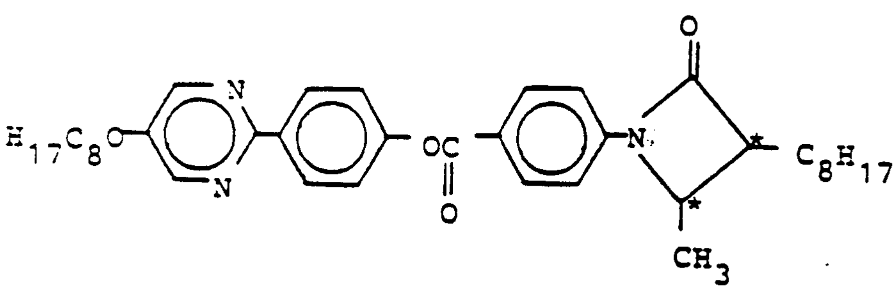

Phasenfolge $X_1$ 93 $X_2$ 102 $S_C$* 121 N* 230 I

Beispiel 5

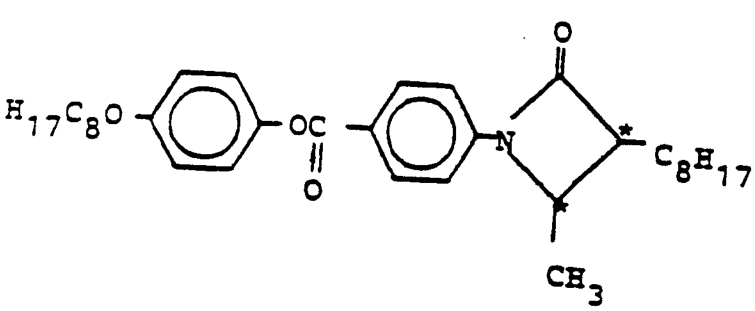

Phasenfolge X 79 $S_C$* 84 $S_A$ 133,7 N* 144,0 I
$[\alpha]_D^{20}$ : 31,9° (c = 1,9 , $CHCl_3$)

Beispiel 6

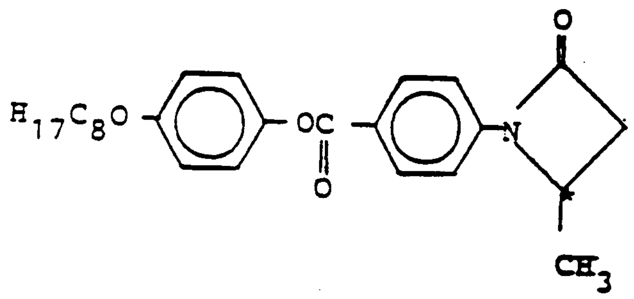

Schmp.: 100°C

Beispiel 7

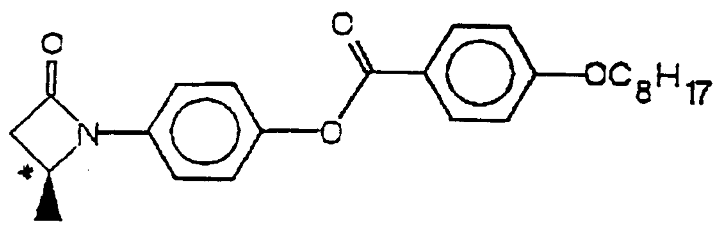

Schmp.: 108°C $[\alpha]_D^{22}$ : + 33,8° (c = 1,1; $CHCl_3$)

Beispiel 8

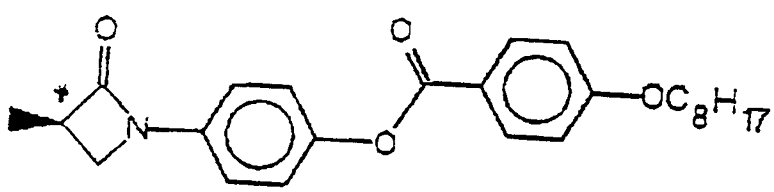

Schmp.: 125°C $[\alpha]_D^{23}$ : + 1,6° (c = 1,2; $CHCl_3$)

Beispiel 9

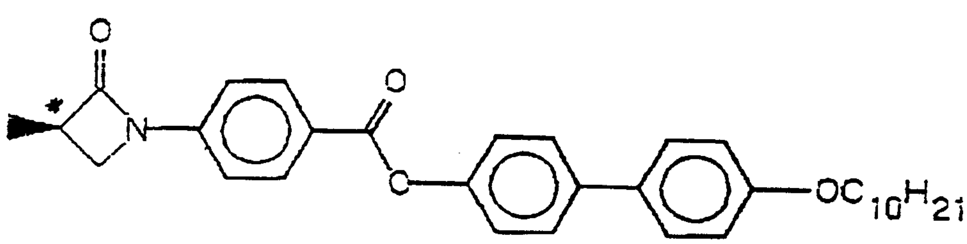

Phasenfolge I 242 $S_x$* 180 X
$[\alpha]_D^{25}$ : + 5,2° (c = 1,2; $CHCl_3$)

Anwendungsbeispiel:

Zur Überprüfung der Eignung der vorstehend beschriebenen Verbindungen als ferroelektrische Dotierstoffe in Flüssigkristall-Systemen mit geneigten smektischen Phasen werden diese zu Testmischungen beigemengt. Zur Messung der Polarisiation und der cholesterischen Verdrillungskraft (N*-HTP) werden die Dotierstoffe in Konzentrationen von jeweils 10 Mol-% zu der unten beschriebenen nicht-chiralen Testmischung M1 zugefügt und die Werte für die spontane Polarisation ($P_s$ in $nC/cm^2$) sowie die cholesterische Verdrillungskraft (HTP in $\mu m^{-1}$) der Mischung bestimmt.

Die $P_s$-Werte werden nach der Methode von H. Diamant et al. (Rev. Sci. Instr., 28, 30, 1957) gemessen. Die Bestimmung der Verdrillungskraft der cholesterischen Phase erfolgt, wie z.B. bei P. Kassubek et al., Mol. Cryst. Liq., Vol. 8, 305-314, 1969 beschrieben, in einer Keilzelle mit Orientierungsschicht durch Ausmessen der Versetzungslinien unter dem Polarisationsmikroskop.

Die Bestimmung der Verdrillungskraft der smektischen C*-Phase erfolgt durch die Methode der selektiven Reflexion. Bei Verwendung einer Zelle mit homöotroper Orientierung des flüssigkristallinen Materials wird zirkular polarisiertes Licht, dessen Wellenlänge und Drehsinn im Flüssigkristall mit der Helixganghöhe bzw. dem - drehsinn übereinstimmt, reflektiert. Da die Verdrillungskraft in der smektischen C*-Phase allgemein mit abnehmender Temperatur steigt, ist die Beobachtung der Temperatur, bei der Licht der Wellenlänge 630 nm reflektiert wird, ein direktes Maß für die HTP der smektischen C*-Phase.

Zur Messung dieser Verdrillungskraft werden die Dotierstoffe in Konzentrationen von 6 % zu der unten beschriebenen chiralen Testmischung M2 zugesetzt. Die chirale Testmischung besitzt bei 6 ° C einen Pitch von + 0,49 $\mu$m. Bei Zugabe eines Dotierstoffes mit umgekehrtem Vorzeichen der HTP sinkt die Verdrillungskraft, so daß die selektive Reflexion im roten Spektralbereich erst bei niedrigeren daß die selektive Reflexion im roten Spektralbereich erst bei niedrigeren Temperaturen beobachtet wird. Je tiefer die Temperatur liegt, desto stärker negativ ist die smektische C*-Verdrillungskraft des Dotierstoffes.

Die nicht-chirale Testmischung M1 besitzt die Zusammensetzung:

| | |
|---|---|
| 5-Octyloxy-2-(4-hexyloxy-phenyl)-pyrimidin | 25,3 Mol-% |
| 5-Octyloxy-2-(4-butyloxy-phenyl)-pyrimidin | 26,7 Mol-% |
| 5-Octyloxy-2-(4-decyloxy-phenyl)-pyrimidin | 21,3 Mol-% |
| 5-Octyloxy-2-(4-octyloxy-phenyl)-pyrimidin | 11,7 Mol-% |
| trans-4-Pentyl-cyclohexancarbonsäure-(4'-(5-decylpyrimidin-2-yl)-phenylester | 15,0 Mol-% |

und zeigt folgende flüssigkristalline Phasenbereiche:
X 9 $S_C$ 84 $S_A$ 93 N 105 I

Die chirale Testmischung M2 besitzt die Zusammensetzung:

| | |
|---|---|
| 5-Octyl-2-(4-octyloxy-phenyl)-pyrimidin | 9,2 Mol-% |
| 5-Octyloxy-2-(4-hexyloxy-phenyl)-pyrimidin | 10,3 Mol-% |
| 5-Octyloxy-2-(4-butyloxy-phenyl)-pyrimidin | 11,3 Mol-% |
| 5-Octyloxy-2-(4-decyloxy-phenyl)-pyrimidin | 6,2 Mol-% |
| 5-Octyloxy-2-(4-octyloxy-phenyl)-pyrimidin | 3,4 Mol-% |
| 5-Octyloxy-2-(4-dodecyloxy-phenyl)-pyrimidin | 6,2 Mol-% |
| trans-4-Pentyl-cyclohexancarbonsäure-(4'-(5-decylpyrimidin-2-yl)phenylester | 14,6 Mol-% |
| Heptansäure-(4-(5-octylpyrimidin-2-yl)) phenylester | 15,3 Mol-% |
| (2S,3S)-2-(4-(5-Octyl-pyrimidin-2-yl)-phenyloxy)-methyl-3-butyl-oxiran | 9,4 Mol-% |
| (2S,3S)-2-(4-(5-Decyl-pyrimidin-2-yl)-phenyloxy)-methyl-3-butyl-oxiran | 4,7 Mol-% |
| (2S,3S)-2-(4-(5-Dodecyl-pyrimidin-2-yl)-phenyloxy)-methyl-3-butyl-oxiran | 9,4 Mol-% |

In Tabelle 1 sind in der 2. und 3. Spalte die Werte für die spontane Polarisation $P_s$ und die Verdrillungskraft N*HTP in der cholesterischen Phase aufgeführt, die sich durch Zusatz von 10 Mol-% eines erfindungsgemäßen Dotierstoffes zu der Mischung M1 ergeben. In Spalte 4 ist die Temperatur angegeben, bei der eine Mischung aus 6 % des erfindungsgemäßen Dotierstoffes und 94 % der Mischung M2 im roten Spektralbereich selektiv reflektiert. In Spalte 4 sind die flüssigkristallinen Phasen des Dotierstoffes aufgeführt. In Tabelle 2 sind zum Vergleich die Werte für $P_s$ und HTP aufgeführt, die sich durch Zusatz von Dotierstoffen mit anderen chiralen Gruppen ergeben. Da $P_s$ und HTP der Dotierstoffe in erster Linie von der chiralen Gruppe abhängen und der mesogene Kern sowie die Länge der Seitenketten nur eine untergeordnete Rolle spielen, ermöglicht Tabelle 2 einen Vergleich mit den Eigenschaften des erfindungsgemäßen Dotierstoffes. Alle Messungen von $P_s$ wurden bei 25 °C durchgeführt, die HTP wurden jeweils ca. 1 °C unterhalb des nematisch-isotropen Phasenübergangs der Mischung gemessen.

Tabelle 1

| erfindungsgemäßer Dotierstoff Struktur | $P_s$ [nC/cm²] | $N^*$-HTP [$\mu m^{-1}$] | Selektiv Reflexion | Phasen |
|---|---|---|---|---|
| 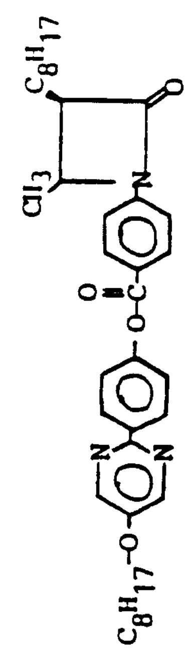 | 26 | - 7,2 | -3°C | X 101 $S_c^*$ 120,8 $N^*$ 230 I |

Tabelle 2

| Vergleichs- beispiel | Struktur | $P_s$[nC/cm²] | N*-HTP [µm⁻¹] | Selektiv Reflexion | Phasen |
|---|---|---|---|---|---|
| 2a | 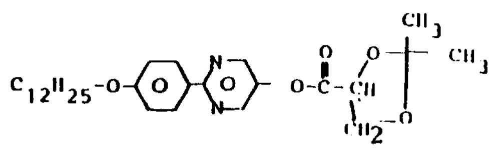 | 18 | - 2,7 | | X 97 I |
| 2b | 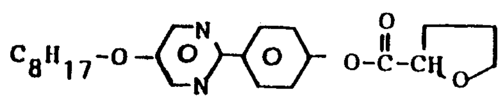 | 17 | + 1,9 | + 2°C | X 87 I |
| 2c | 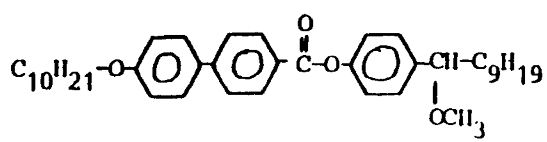 | < 1 | 0 | | X 40 $S_C^*$ 82 $S_A^*$ 108 I |

| Vergleichsbeispiel | Struktur | $P_s$ [nC/cm²] | $N^*$-HTP [µm⁻¹] | Selektiv Reflexion | Phasen |
|---|---|---|---|---|---|
| 2d | 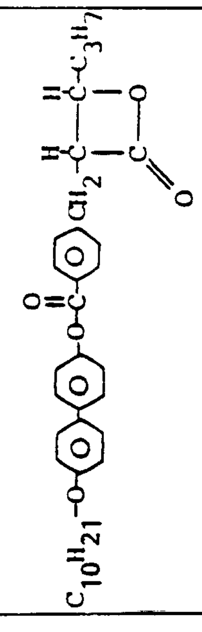 | < 1 | 4,3 | | X 135 BP 127 I |

Ein Vergleich der Tabellen zeigt, daß der erfindungsgemäße Dotierstoff eine überraschend große Polarisation induziert. Durch Verwendung von Mischungen mit derartigen Dotierstoffen lassen sich daher besonders schnell schaltende Flüssigkristallsysteme erzeugen. Die große Verdrillungskraft des erfindungsgemäßen Dotierstoffs kann dazu genutzt werden, um die Helix einer chiralen Flüssigkristallmischung zu kompensieren oder aber einen kurzen $S_C$-Pitch zu erzeugen.

Desweiteren zeigt ein Vergleich der Tabellen, daß der erfindungsgemäße Dotierstoff im Gegensatz zu einigen Vergleichsbeispielen in Tabelle 2 eine smektische C-Phase sowie eine nematische Phase besitzt und dadurch den flüssigkristallinen Phasenbereich einer Mischung günstig beeinflußt.

## Patentansprüche

**1.** Azetidinone der allgemeinen Formel I

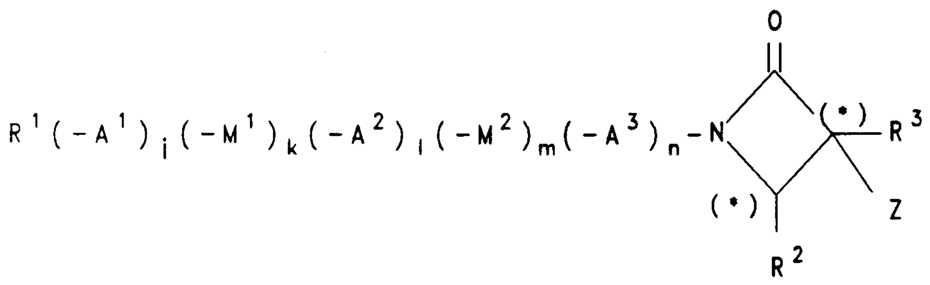

(I)

in der die Symbole und Indices folgende Bedeutung haben:

(*) ist gegebenenfalls ein chirales Zentrum

$R^1$ ist ein geradkettiger oder verzweigter Alkylrest mit 1 bis 16 C-Atomen oder ein geradkettiger oder verzweigter Alkenylrest mit 3 bis 16 C-Atomen, in dem asymmetrische C-Atome enthalten sein können, eine oder mehrere nicht benachbarte $-CH_2$-Gruppen durch -O-, -S-, -CO-, -O-CO- und/oder - CO-O-ersetzt sein können, wodurch sich die Zahl der C-Atome in dem Rest entsprechend reduziert, und in dem ein oder mehrere H durch F, Cl, Br oder CN ersetzt sein können,

$R^2$ ist ein Wasserstoffatom oder ein Alkylrest mit 1 bis 10 C-Atomen, in dem auch die dem Ring benachbarte $-CH_2$-Gruppe durch -O- oder

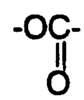

ersetzt sein kann, wodurch sich die Zahl der C-Atome in dem Rest entsprechend reduziert,

$R^3$ ist ein Wasserstoffatom oder ein Alkylrest mit 1 bis 10 C-Atomen

Z bedeutet H, F, Cl, $-NO_2$ oder $-N_3$

j und l sind 0, 1 oder 2,

k und m sind 0 oder 1,

n ist 0, 1 oder 2,

mit folgender Maßgabe: wenn j und/oder l = null sind, ist k = null; wenn n = null ist, ist m = null; die Summe j + l + n ist minimal 1 und maximal 3,

$-A^1$, $-A^2$ bedeutet

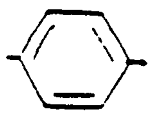, 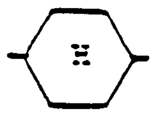, 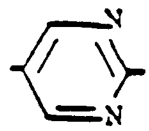, 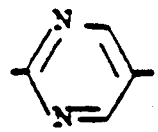, 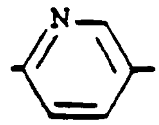,

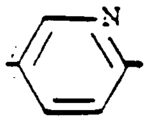, 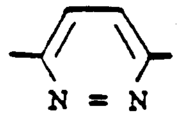, 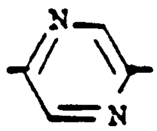, 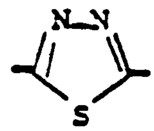, 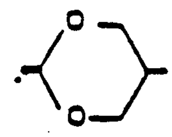,

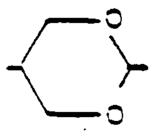, 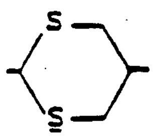, 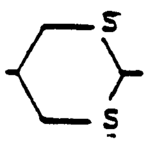, 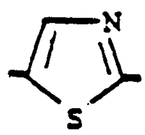, 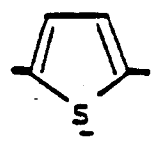,

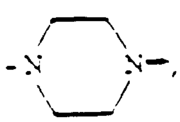, 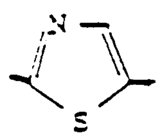, 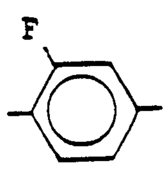, 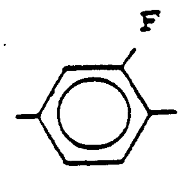, 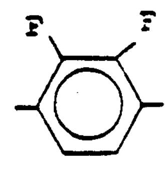,

$-A^3$ bedeutet

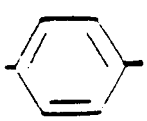, 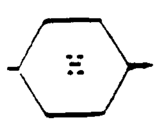, 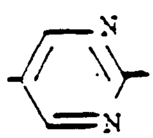, 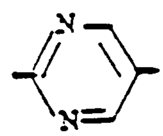, 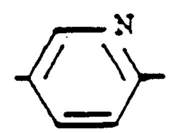,

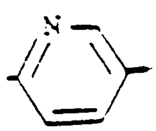, 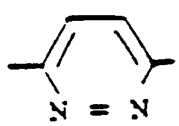, 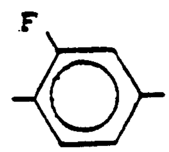, 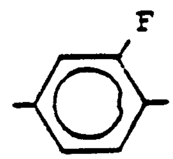, 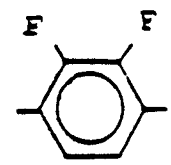,

$-M^1$, $-M^2$ ist -O-CO-, -CO-O-, $-CH_2CH_2-$, -CH = CH-, $-CH_2O$ oder $-OCH_2$,

**2.** Azetidinone nach Anspruch 1, dadurch gekennzeichnet, daß

$R^1$ ein geradkettiger oder Verzweigter Alkyl- oder Alkenylrest mit 6 bis 12 C-Atomen ist, der ein asymmetrisches C-Atom enthalten kann, und in dem eine $CH_2$-Gruppe durch -O-, -CO-, oder -COO- ersetzt sein kann, wodurch sich die Zahl der C-Atome in dem Rest entsprechend reduziert, und

die Gruppierung $(-A^1)_j(-M^1)_k(-A^2)_l(-M^2)_m(-A^3)_n-$ folgende Bedeutung hat:

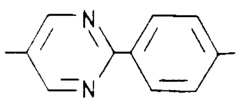 , 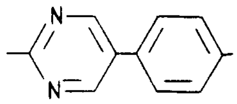 , 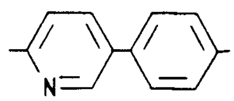 , 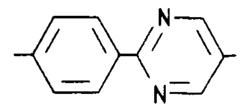

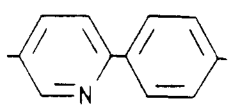 , 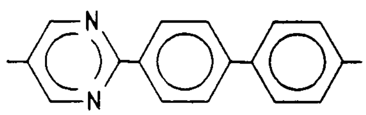 , 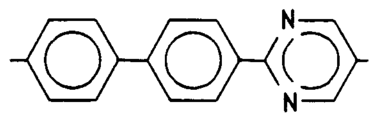 ,

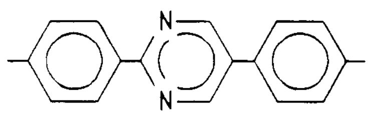 , 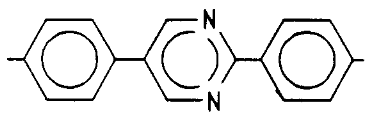 ,

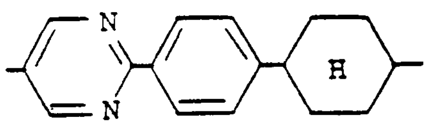 , 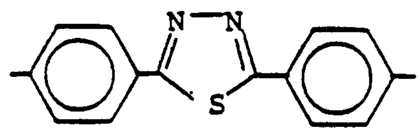

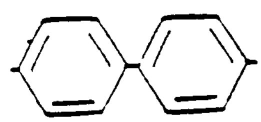 ,

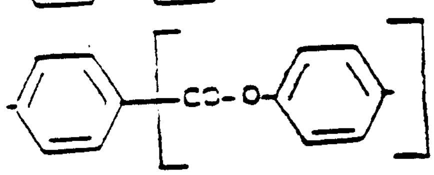 1 oder 2 , 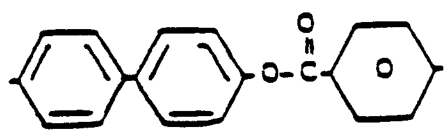

3. Flüssigkristallmischungen, gekennzeichnet durch einen Gehalt von 0,01 bis 60 Gew.-% an mindestens einem Azetidinon nach Anspruch 1.

4. Ferroelektrische Flüssigkristallmischungen, gekennzeichnet durch einen Gehalt von 0,01 bis 60 Gew.-% an mindestens einem Azetidinon nach Anspruch 1.

5. Elektrooptische Schalt- und Anzeigeelemente, die eine Flüssigkristallmischung nach Anspruch 3 oder 4 enthalten.

Europäisches Patentamt

# EUROPÄISCHER RECHERCHENBERICHT

Nummer der Anmeldung

EP 92 11 0132

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | JAPANESE JOURNAL OF APPLIED PHYSICS<br>Bd. 27, Nr. 12, 1988, TOKYO JP<br>Seiten L2241 - L2244<br>H.R.DÜBAL 'three Classes of New Chiral Dopants: Synthesis and Physical Qualification as Dopants for Practical FLC-Mixtures'<br>* das ganze Dokument *<br>----- | 1,3-5 | C07D205/08<br>C09K19/58<br>G02F1/13 |
| | | | **RECHERCHIERTE SACHGEBIETE (Int. Cl.5 )** |
| | | | C07D<br>C09K |

Der vorliegende Recherchenbericht wurde für alle Patentansprüche erstellt

| Recherchenort | Abschlußdatum der Recherche | Prüfer |
|---|---|---|
| DEN HAAG | 28 SEPTEMBER 1992 | BESLIER L.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)